# EUROPÄISCHE PATENTANMELDUNG

(11) **EP 2 944 348 A1**
(43) Veröffentlichungstag der Anmeldung: **18.11.2015**
(21) Anmeldenummer: 15020072.3
(22) Anmeldetag: 11.05.2015
(51) Int. Cl.: A61M 37/00

(54) **KAPILLARSTECHER**

(30) Priorität: 13.05.2014 DE 102014006846
(71) Anmelder: Blank, Anton, 70173 Stuttgart (DE)
(72) Erfinder: Blank, Anton, 70173 Stuttgart (DE)

(57) **Zusammenfassung**

Vorrichtung zum repetitiven Einstechen von Stoffen in eine organische Haut, mit einem Gehäuse (2), das durch einen Antrieb (1) in repetitive Hubbewegungen versetzt wird und mit mindestens einem Kapillarstecher (4), der mit dem Gehäuse (2) fest verbunden ist, wobei der mindestens eine Kapillarstecher (4) konfiguriert ist, einen Stoff (6) mittels mindestens einer Kapillarkraft durch den mindestens einen Kapillarstecher (4) bis zu dessen Stechspitze (3) zu transportieren, die Stechspitze (3) mit dem Stoff (6) wenigstens teilweise zu benetzen und den Stoff (6) in eine organische Haut einzustechen.

## Beschreibung

Die Erfindung betrifft einen Kapillarstecher zum Einstechen von Stoffen in eine organische Haut und/oder zur Stimulation einer organischen Haut.

Diese Anmeldung kann einen Gegenstand enthalten, der mit einem oder mehreren der in folgenden Dokumenten des Anmelders offenbarten oder beanspruchten Gegenstände verwandt ist: EP000002671609 und EP000002671610.

### Stand der Technik

Vorrichtungen zum repetitiven Einstechen in eine organische Haut werden beispielsweise zum dekorativen Einbringen von Farben für Permanent Make-up (PMU) oder Tätowierungen (Tattoo) oder zum Einbringen kosmetischer oder medizinischer Stoffe (Meso-Therapie) oder zur Hautstimulation (Regenerations-Provokation) unter Verwendung von beispielsweise Gleitstoffen und/oder die Heilung begünstigender Stoffe genutzt. Die Vorrichtungen bestehen üblicherweise aus einer Antriebseinheit (Handgerät), das eine repetitive Hubbewegung auf ein vom Handgerät lösbares, mit dem einzubringenden Stoff benetztes Stechelement überträgt, um die Haut bei jedem Hub anzustechen, damit der Stoff in die Haut eindringen und in ihr verbleiben kann und/oder das Einstechen ist zur Stimulation der Haut vorgesehen, um durch kleine Verletzungen eine Regeneration der Haut zu provozieren.

Das Einbringen der Stoffe oder das Einstechen zur Hautstimulation in eine vom Anwender zugeordnete, also vorbestimmbare sowie genaue Tiefe der Haut wird in der Praxis bisher vernachlässigt, obwohl bekannt ist, in welche optimalen Tiefen (Hautschichten) die jeweiligen Stoffe in Abhängigkeit von der jeweiligen Anwendung eingebracht werden müssen oder in welcher Hauttiefe eine wirkungsvolle Stimulation erfolgen muss. Bisher bleibt es dem Anwender überlassen, eine Einstechtiefe im untersten Millimeterbereich auf weniger als einen Zehntel-Millimeter genau freihändig bereit zu stellen und diese genaue Tiefe mit der Arbeitsbewegung seiner Hand über eine Strecke von mehreren Zentimetern oder eine Fläche von mehreren Quadratzentimetern beizubehalten, was vom Anwender nicht durchführbar ist, da die tatsächliche Einstechtiefe während der Anwendung weder sichtbar noch messbar ist.

Das Dokument EP2682146 eröffnete eine Vorrichtung mit einstellbarer Stechtiefe an einem Handgerät, dessen Antriebshub unveränderbar ist und dessen Nadel sowie dessen Stechtiefeneinsteller über einen Antriebsstößel synchron mitbewegt werden. Die Stechtiefe wird dadurch eingestellt, dass der Abstand eines vorderen Endes einer Nadeldüse durch Verlagern relativ zur Nadelspitze (distales Ende) verändert wird und damit, wie schon früher in EP000002671609 eröffnet, das vordere Ende der Nadeldüse als Anschlag auf der Haut fungiert und dadurch die Nadel nur soweit in die Haut eindringen kann, wie sie über das Ende der Nadeldüse hinaus übersteht. Diese Anordnung hat den entscheidenden Nachteil, dass der zur exakten Tiefeneinstellung notwendige Nullpunkt der Nadel weder herstellerseitig noch durch den Anwender bestimmt werden kann, weil sämtliche Komponenten fertigungsbedingt Toleranzen aufweisen und daher die für jede Anwendung auszutauschenden unterschiedlich langen Komponenten jeweils auf ein Handgerät mit gleichfalls unterschiedlicher Länge treffen. Zur Verdeutlichung der Größenordnung: Die am häufigsten in der Praxis vorkommenden Anwendungen erfordern Einbringtiefen von 0,05mm bis 0,3mm in eine organische Hautschicht (Dermis). Die Eichung des Nullpunkts der Nadelspitze relativ zum Ende der Nadeldüse müsste für eine praxisnahe Genauigkeit deutlich kleiner als 0,05mm (50µm) sein und könnte letztlich nur vom Anwender direkt durchgeführt werden, doch dieser kleine Wert ist selbst mit einer Lupe von einem Menschen nicht einstellbar. Mit dieser Vorrichtung lässt sich zwar die Einstechtiefe variieren, doch eine exakt definierte Tiefe kann damit im erforderlichen Tiefenbereich nicht erreicht werden, so dass weiterhin nur in meist zu großen und für den Endkunden schädlichen Tiefen gearbeitet werden kann.

Nach dem Stand der Technik, der teilweise durch die beiden Patentanmeldungen des Anmelders nach [0002] repräsentiert ist, wird die in [0005] genannte Toleranzproblematik erfinderisch dadurch gelöst, dass nur noch ein einziges Bauteil, nämlich die Stechspitze, für die Genauigkeit der Tiefe herangezogen wird. Die Fertigung von Spitzenlängen auch im untersten erforderlichen Längenbereich, von 50µm und eine Längengenauigkeit von wenigen µm sind heute problemlos möglich.

### Die Erfindung

Aufgabe der Erfindung ist es, eine Vorrichtung zu schaffen, welche die in [0002] offenbarten Vorrichtungen in Bezug auf die Einbringbarkeit von Stoffen durch die Kapillareffekte und die Eigenschaften der Stechspitze für ein anwendungsbezogenes und stoffbezogenes Einstechen in eine organische Haut verbessern.

Diese Aufgabe wird erfindungsgemäß mit einer Vorrichtung nach dem Oberbegriff des unabhängigen Anspruchs 1 dadurch gelöst, dass die Kapillarstecher 4 in ihrem Inneren frei für einen Stoff durchgängig sind und deren Stechspitzen 3 entweder direkt in das Kapillarmaterial eingearbeitet sind oder aus separat gefertigten Stechspitzen, die nachträglich befestigt werden, bestehen.

Ein wesentlicher Vorteil, welcher mit der Erfindung gegenüber dem Stand der Technik erreicht wird, besteht darin, dass entsprechend dem Stand der Technik, die von Stechmittel zu Stechmittel unterschiedlichen Kapillarkräfte, die durch das bisher darin befindliche Stechmittel (Nadel) hervorgerufen wurden, weil die Nadel bei der Fertigung jeweils nicht zentrisch sondern jeweils auch eine unterschiedliche Schräglage in der Kapillare einnahm, beseitigt wird. Die Kapillarkräfte können deshalb nun (ohne innenliegende Nadel) bei der Fertigung wesentlich genauer für die jeweilige Anwendung eingestellt und im Fertigungsprozess konstant gehalten werden.

Ein wesentlicher Vorteil der Ausgestaltung der Erfindung wird erfindungsgemäß dadurch erreicht, dass die Stechspitzen durch direktes bearbeiten eines Endes einer Kapillare gebildet und damit verschiedene, für spezifische Anwendungen vorteilhafte Spitzenformen direkt an der Kapillare hergestellt werden. Damit geht auch eine einfachere und preiswertere Fertigung des Kapillarstechers einher.

Ein weiterer Vorteil der Erfindung gegenüber dem Stand der Technik besteht darin, dass bei von den Kapillaren getrennt gefertigten Stechspitzen (Stechspitzenelemente) weitere und über die nach hinausgehenden, für spezifische Anwendungen vorteilhafte Spitzenformen realisiert werden können, die nach dem Stand der Technik nicht möglich wären. Derartig separat gefertigte Stechspitzen werden in einem anschließenden Fertigungsschritt an der Kapillare befestigt.

Ein weiterer wesentlicher Vorteil der Erfindung gegenüber dem Stand der Technik besteht darin, dass durch eine mindestens zweistufige Nutzung des Kapillareffekts das Einstechen des Stoffes in eine organische Haut nochmals verbessert wird.

### Beschreibung bevorzugter Ausführungsformen der Erfindung

Die Erfindung wird nachfolgend anhand von Ausführungsbeispielen unter Bezugnahme auf Zeichnungen näher erläutert. Hierbei zeigen
- Fig. 1: eine schematische Darstellung eines Stechelements mit einem Kapillarstecher,
- Fig. 2a bis 2d: schematische Darstellungen von Spitzenformen, die durch direktes Bearbeiten eines Kapillarendes geschaffen sind, um einen Kapillarstecher zu bilden,
- Fig. 3a bis 3d: schematische Darstellungen von Spitzenformen die separat hergestellt werden und in einem anschließenden Fertigungsschritt an oder in der Kapillare befestigt werden, um einen Kapillarstecher zu bilden,
- Fig. 4a und 4b: schematische Darstellungen von Stechelementen mit mehreren Kapillarstechern in zwei unterschiedlichen Anordnungen.

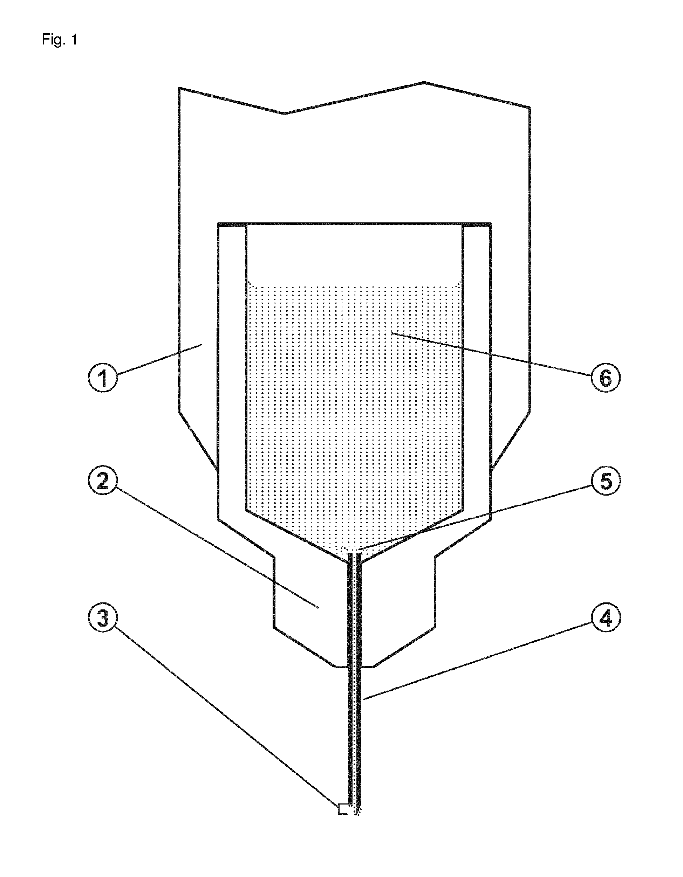

Fig. 1 zeigt eine Vorrichtung eines erfindungsgemäßen Kapillarstechers zum Einbringen von Stoffen in eine organische Haut, mit einem Kapillarstecher 4, der mit einem Gehäuse 2 fest verbunden ist und dessen distales Ende (Behandlungsende) eine Stechspitze 3 aufweist, die durch direktes Bearbeiten einer Kapillare (Kapillarröhrchen) gebildet ist. Das Gehäuse 2 koppelt zusammen mit dem darin fest eingebauten Kapillarstecher 4 lösbar mit dem Antrieb 1, der eine repetitive Hubbewegung zum Einstechen der Stechspitze 3 in eine organische Haut bereitstellt. Der Stoff 6 dringt unterstützt durch den Kapillareffekt des Kapillarstechers 4 durch das offene Ende 5 des Kapillarstechers 4 in die Kapillare ein. Bei jedem Einstich der Stechspitze 3 in eine Haut verbleibt eine kleine Menge an Stoff in der Haut. Wird kein Stoff 6 verwendet, so erfolgt das Einstechen in die Haut analog wie oben beschrieben, die Haut wird lediglich definiert verletzt, was sie zur Regeneration anregt.

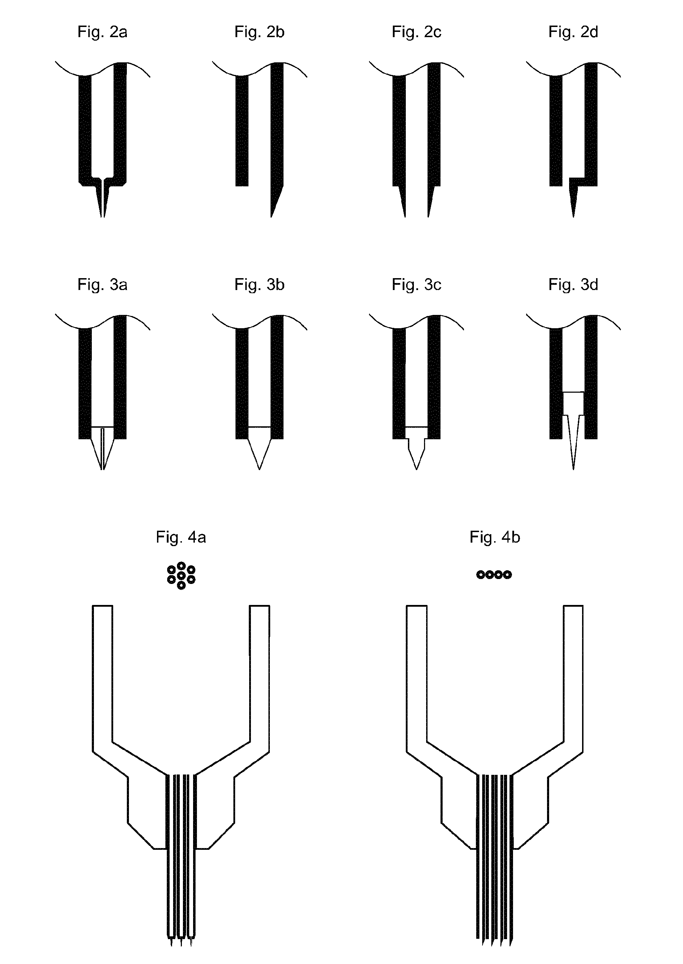

Die Fig. 2a und 3a zeigen beispielhaft je einen im Sinne der Erfindung besonders vorteilhaften Kapillarstecher unter 2-stufiger Ausnutzung des Kapillareffekts. Der Stoff wird durch den Kapillareffekt des Kapillarröhrchens bis zur Stechspitze befördert und dann durch den weiteren Kapillareffekt der geteilten Stechspitze bis zu deren Spitze transportiert. Damit ist der Kapillarspalt der Stechspitze immer bis zu seinem vordersten Ende mit dem Stoff benetzt, der Stoff kann dadurch ohne größere Abstreifverluste entsprechend der vollen Einstechtiefe in eine Haut eindringen.

Die Fig. 2a bis 2d zeigen beispielhaft verschiedene Spitzenformen die aus der Kapillare direkt heraus gearbeitet sind. Dabei sind beispielsweise die Fig. 2b und 2d optimiert, um ein Zusetzten des Kapillarstechers mit Hautresten entgegen zu wirken, die Fig. 2a und 2d führen die Stechspitze in das Zentrum der Kapillare, damit sie vom Stoff optimal benetzt wird und die Fig. 2c zeigt eine Zweifachspitze, die pro Hub eine größere Menge an Stoff in eine organische Haut einstechen kann. Die Vorteile eines unbehinderten und definierbaren Transports des Stoffes durch die Kapillare und die Möglichkeit der Ausbildung vorteilhafter Spitzenformen der Stechspitze werden deutlich.

Die Fig. 3a bis 3d zeigen beispielhaft verschiedene Spitzenformen die zunächst separat angefertigt werden, um dann in einem anschließenden Fertigungsschritt an oder in der Kapillare befestigt werden, um einen Kapillarstecher zu bilden. Die Vorteile einer Ausbildung jeweils zentrierter, nach [0015] teilweise nicht herstellbarer Spitzenformen der Stechspitze werden deutlich.

Fig. 4a+4b zeigen beispielhaft Anordnungen von Stechelementen aus mehreren Kapillarstechern. Der Anzahl der Kapillarstecher und der Anordnung derselben sind kaum Grenzen gesetzt.

Die in der vorstehenden Beschreibung, der Zeichnung und den Ansprüchen offenbarte Merkmale der Erfindung können sowohl einzeln als auch in beliebiger Kombination für die Verwirklichung der Erfindung in ihren verschiedenen Ausführungsformen von Bedeutung sein.

## Patentansprüche

1. Vorrichtung zum repetitiven Einstechen von Stoffen in eine organische Haut, mit:
- einem Gehäuse (2), das durch einen Antrieb (1) in repetitive Hubbewegungen versetzt wird,
- einem oder mehreren Kapillarstechern (4), die mit dem Gehäuse (2) fest verbunden sind,
- und einem Stoff (6),
**dadurch gekennzeichnet, dass** einer oder mehrere Kapillarstecher (4) konfiguriert sind, einen Stoff (6) unter Nutzung einer oder mehrerer Kapillarkräfte (Kapillareffekt) durch den einen oder die mehreren Kapillarstecher (4) hindurch bis zu deren Stechspitze (3) zu transportieren, die Stechspitze (3) mit dem Stoff (6) wenigstens teilweise zu benetzen und den Stoff (6) mit der Stechspitz (3) in eine organische Haut einzustechen.

2. Vorrichtung nach Anspruch 1, **dadurch gekennzeichnet, dass** mit dem Innendurchmesser und der Länge des einen oder der mehreren Kapillarstecher (4) schon während der Fertigung die Kapillarkräfte des oder der Kapillarstecher (4) derart gesteuert werden, dass das Einstechen eines Stoffes (6) mit der Stechspitze (3) in eine organische Haut für den jeweiligen Anwendungsfall und in Bezug auf die jeweiligen physikalischen, kosmetischen und medizinischen Eigenschaften des Stoffes (6) verbessert sind.

3. Vorrichtung nach Anspruch 1, **dadurch gekennzeichnet, dass** die Stechspitze oder die Stechspitzen (3) des einen oder der mehreren Kapillarstecher (4) direkt aus einem Kapillarröhrchen gebildet sind.

4. Vorrichtung nach Anspruch 1, **dadurch gekennzeichnet, dass** die Stechspitze oder die Stechspitzen (3) des einen oder der mehreren Kapillarstecher (4) separat gefertigte Komponenten sind, die an oder in einem Kapillarröhrchen befestigt werden, um Kapillarstecher zu bilden.

5. Vorrichtung nach Anspruch 3 und 4, **dadurch gekennzeichnet, dass** die Form und/oder die Position der einen oder der mehreren Stechspitzen (3) an dem einen oder den mehreren Kapillarstechern (4) derart geformt und/oder positioniert sind, dass das Einstechen eines Wirkstoffes (6) in eine organische Haut im jeweiligen Anwendungsfall und in Bezug auf den jeweiligen Stoff (6) verbessert sind.

6. Verwendung einer Vorrichtung nach mindestens einem der vorangegangenen Ansprüche zum Einstechen von Stoffen und/oder zur Stimulation einer organischen Haut.
